Europäisches Patentamt

(19) European Patent Office

Office européen des brevets

(11) Publication number: 0 114 615
A2

(12) **EUROPEAN PATENT APPLICATION**

(21) Application number: 84100250.4

(22) Date of filing: 12.01.84

(51) Int. Cl.³: **G 01 N 33/74**
**G 01 N 33/54**

(30) Priority: 25.01.83 US 460734

(43) Date of publication of application:
01.08.84 Bulletin 84/31

(84) Designated Contracting States:
BE DE FR GB IT

(71) Applicant: ABBOTT LABORATORIES
14th Street and Sheridan Road
North Chicago, Illinois 60064(US)

(72) Inventor: March, Steven Carl
1307 Winchester Road
Libertyville, I11. 60048(US)

(72) Inventor: Shipchandler, Mohammed Tyebji
640 Burdick
Libertyville, I11. 60048(IN)

(74) Representative: Modiano, Guido et al,
MODIANO, JOSIF, PISANTY & STAUB Modiano &
Associati Via Meravigli, 16
I-20123 Milan(IT)

(54) Alkaline phosphatase labeled steroid hormone glucoronides.

(57) The present invention relates to a novel class of enzyme labeled steroid hormone glucuronides useful as tracers in enzyme immunoassays. More specifically, the present invention provides a class of tracers comprising steroid hormone glucuronides covalently attached to alkaline phosphatase and characterized by substantially possessing the enzymatic activity of alkaline phosphatase alone and the immunological activity of steroid hormone glucuronide alone.

EP 0 114 615 A2

ALKALINE PHOSPHATASE LABELED STEROID HORMONE GLUCURONIDES

The present invention relates to a novel class of enzyme labeled steroid hormone glucuronides useful as tracers in enzyme immunoassays. More specifically, the present invention provides a class of tracers comprising steroid hormone glucuronides covalently attached to alkaline phosphatase and characterized by substantially possessing the enzymatic activity of alkaline phosphatase alone and the immunological activity of steroid hormone glucuronide alone.

Steroid hormones are normally synthesized from cholesterol by organisms. The principal vertebrate steroid hormones and their physiological effects are: croticosteroids -- mineral balance, $Na^+$ retention, metabolism and gluconeogenesis; progesterone -- proliferation of the uterine mucosa (secretory phase); estradiol -- proliferation of the uterine mucosa (strus); and testosterone -- maintenance of the accessory glands of the genital tract and secondary sex characteristics. See, Karlson, *Introduction to Modern Biochemistry*, Academic Press, New York, New York (3rd Edition, 1971), page 358. It is generally recognized that in humans endogenous steroid hormones are excreted in urine principally as water-soluble glucuronide derivatives. See, Kellie, "The Radioimmunoassay of Steroid Conjugates", *J. Steroid Biochemistry*, 6, page 277 (1975).

The important physiological responses elicited by steroid hormones have prompted substantial research into methods for the quantitative determination of these hormones and their glucuronide derivatives. Particular attention has been directed towards the relationship between endogenous concentrations of the so-called female sex hormones (estradiol, progesterone) and their role in the human female menstrual cycle. A recent report suggests that determination of estradiol glucuronide levels in a sample may be an indication of ovulation and that the occurrence of ovulation may be confirmed by determining pregnanediol glucuronide levels.

See, Stanczyk, et al., "Direct Radioimmunoassay of Urinary Estrogen and Pregnanediol Glucuronides During the Menstrual Cycle", *Am. J. Obstet. Gynecol.*, 137, page 443 (1980). Monitoring of urinary estradiol glucuronide levels, plasma estradiol levels and serum progesterone levels has also been reported as helpful in the treatment of human infertility. See, Black, et al, "An Assessment of Urinary and Plasma Steroid Estimations for Monitoring Treatment of Anovulation With Gonadotropins", *J. Obstet. Gynec. British Commonwealth*, 81, page 667 (1974).

Several radioimmunoassays for determining steroid hormone and steroid hormone glucuronide concentrations have been reported. See, Kellie, supra, page 277; van Weeman, et al, "Enzyme-Immunoassay of Steroid Hormones: Possibilities and Pitfalls", *J. Steroid Biochemistry*, 11, page 147 (1979). (1979). An advantage of radioimmunoassays relative to biological or chemical assays is the high degree of sensitivity obtainable with radiolabelled compounds. Steroid hormones may occur in human body fluids in concentrations ranging from picomoles per liter (estradiol, early leuteal phase of menstrual cycle) to 100 micromoles per liter (urinary estriol at end of pregnancy). Radioimmunoassays have the capacity to detect steroid hormones at picomole per liter concentrations, well beyond the sensitivity of biological or chemical assay systems. See, van Weeman, et al, *supra*, page 147.

Problems associated with radioimmunoassays include the use of radiolabeled compounds which require specially equipped laboratories, specially trained personnel, and expensive counting equipment. In addition, some radioisotopes also have relatively short half-lives. Furthermore, waste disposal and the need for special licenses are also potential problems. See, van Weeman, et al, *supra*, page 147; Kohen, et al, "Nonradioisotopic Homogeneous Steroid Immunoassays", *J. Steroid Biochemistry*, 11, page 161 (1979).

Radioimmunoassays also require a significant amount of time to complete. One recently-reported "ligand" radioimmunoassay for steroid hormone glucuronides requires a 2-1/2 hour incubation period before the radioactivity counting procedure may be commenced. See, Chatterton, et al., "Radioimmunoassay of Pregnanediol Concentrations in Early Morning Urine Specimens For Assessment Of Leuteal Function In Women", *Fertility and Sterility*, 37, pages 361-363 (1982). Labeling of steroid hormones with a gamma emitter, $I^{125}$, may also reduce immunoreactivity of the steroid hormone. See, Jeaffcoate, "Progress Toward The Wider Use Of Steroid Immunoassays", *J. Steroid Biochemistry*, page 1051 (1979). Radioimmunoassays of serum steroid hormone necessarily requires repeated venipuncture of the subject and should be performed by trained medical personnel.

The use of nonisotopic labeled compounds in an immunoassay system has been proposed to avoid many of the problems inherent in radioimmunoassays. Assays for steroid hormones utilizing nonradioactive labeled compounds include so-called labeled ligand assays wherein determination of the steroid hormone consists of measuring the biological, optical, or chemical activity of fluorophores, bacteriophages, coenzymes or enzymes. See, van Weeman, et al, *supra*, page 147. Assays utilizing enzyme activity to quantify steroid hormones, commonly referred to as enzyme immunoassays or enzyme-linked immunoassays, have been reported for estrogens, progesterone, testosterone and cortisol.

Recently developed steroid hormone enzyme immunoassays exhibit sensitivities equal or near to those of radioimmunoassays. See, Numazawa, et al, "Picogram Order Enzyme Immunoassay of Oestradiol", *FEBS Letter*, 79, pages 396-397, (1977); Bosch, et al, "Enzyme Immunoassays For Total Oestrogens In Pregnancy Plasma Or Serum", *Clin. Chem. Acta*, 89, pages 59, 63 (1979). The enzymes conjugated to steroid hormones and utilized in enzyme immunoassays include horseradish peroxidase, β-D-galactosidase, glucoamylase, alkaline phosphatase, glucose-6-phosphatase and malate dehydrogenase.

These have been attempts to prepare enzyme-labeled steroid hormone glucuronides which retain both the enzymatic and the immunological activities of the starting materials. See Rajkowski, et al., "The Efficiency of Different Coupling Procedures for the Linkage of Oestriol-16α-Glucuronide, Oestrone-3-Glucuronide and Pregnanediol-3α-Glucuronide to Four Different Enzymes", *J. Steroid Biochemistry*, 14, pages 861-865 (1981). The standard procedure used to prepare steroid hormone glucuronide immunogens comprises formation of peptide bonds between the carboxylic acid group of the glucuronide, and the ε-aminolysine groups of enzyme amino acid. In the study, estradiol glucuronide was coupled by its glucuronide carboxylic acid function to one of four enzymes (horseradish peroxidase, urease, alcohol dehydrogenase, or glucose-6-phosphate dehydrogenase) by each of the following methods: (1) a carbodiimide method using 1-ethyl-3(3-dimethylaminopropyl)carbodiimide·HCL: (2) a carbodiimide method using 1-cyclohexyl-3-3(2-morpholinoethyl).carbodiimidemethyl-p-toluenesulphonate; (3) the Erlanger mixed anhydride method; and (4) the hydroxysuccinimide / N,N'dicyclohexylcarbodiimide method. Molar ratios of 3 or 20 estradiol glucuronide to 1 of enzyme were utilized. Only the mixed anhydride method produced an enzyme-labelled product which retained any measurable enzyme activity. Subsequent attempts were made using the mixed anhydride method to couple preganediol glucuronide, estradiol glucuronide, and estriol glucuronide to each of the four previously-mentioned enzymes. The reported results indicated that glucose-6-phosphatase, alcohol dehydrogenase, and urease all suffered heavy losses of their specific enzymatic activity. Only horseradish peroxidase retained more than 50% of its enzymatic activity when coupled with each of the three glucuronides. See, Rajkowski, et al, *supra*, pages 861-865. Problems are associated with the use of horseradish peroxidase as an enzyme label, include loss of enzymatic activity upon exposure to light, air or moisture. Horseradish peroxidase also must be stored in liquid form, thus, increasing problems of contamination and decreasing shelf life.

Although alkaline phosphatase is noted for high stability and has been commercially used as an antibody label in immunoassay systems, no enzyme-labeled compounds have been reported in which alkaline phosphatase has been covalently attached to steroid hormone glucuronides. See, McComb, et al, *Alkaline Phosphatase*, Plenum Press, New York, New York, (1979). One explanation is the report of nearly total loss of alkaline phosphatase's enzymatic activity when labeled with other small molecular weight compounds. See, Guedson, "The Use of Avidin-Biotin Interaction in Immuno-enzymatic Techniques", *J. Histochem. Cytochem*, **22**, page 1131, (1979). Another explanation may be that the conventional methods of coupling alkaline phosphatase to steroid hormone glucuronides produce extensive cross-linking of the enzyme to itself between carobyxlic acid and amine side groups of the enzyme's amino acids, resutling in deactivation of the enzyme.

It is an object of the present invention to provide a class of enzyme-labeled steroid hormone glucuronides which are both enzymatically and immunologically stable.

## Summary Of The Invention

The present invention provides a class of novel enzyme-labeled steroid hormone glucuronides useful as tracers in enzyme immunoassays and methods for preparing such glucuronides. More specifically, the present invention provides a class of alkaline phosphase-labeled steroid hormone glucuronide tracers comprising a steroid hormone glucuronide covalently bound to alkaline phosphatase, characterized by substantially possessing the enzymatic activity of alkaline phosphatase alone and the immunological activity of the steroid hormone alone. The present invention provides a novel method for preparing alkaline phosphatase labeled glucuronide tracers through esterification of a steroid hormone glucuronide and subsequent formation of amide bonds between the esterified steroid hormone glucuronide and alkaline phosphatase.

## Detailed Description Of The Invention

The tracers of the present invention are characterized in that the free carboxyl group of a steroid hormone glucuronide is esterified to form a activated steroid hormone glucuronide ester and the activated ester is treated with alkaline phosphatase so as to form an amide bond between the amine group of alkaline phosphatase and the carboxyl group of the steroid hormone glucuronides. Preferred tracers according to the invention include preganediol glucuronide, estradiol glucuronide, estriol glucuronide, or esterone glucuronide covalently bound to alkaline phosphatase as described above.

According to a preferred embodiment of the method of the invention, an N-hydroxysuccinimide ester of pregnanediol glucuronide is prepared by mixing pregnanediol glucuonide with N-hydroxysuccinimide in the presence of 1-ethyl-3(3-dimethylaminopropyl)carbodiimide hydrochloride in dimethyl formamide. The pregnanediol glucuronide ester thus prepared is reacted with alkaline phosphatase in an appropriate buffer at molar ratios of glucuronide to alkaline phosphatase in the range of from about 500:1 to about 100:1, preferably from 200:1 to 100:1. The alkaline phosphatase labeled pregnanediol glucuronide produce is purified by gel chromatography or dialysis. Other alkaline phosphatase labeled glucuronides may be prepared by utilizing other glucuronides such as for example, estradiol glucuronide, estriol glucuronide or esterone glucuronide, in lieu of pregnanediol glucuronide in the above-described procedure.

The reagents and materials employed in the methods of the present invention are generally commercially available or readily prepared by one of ordinary skill in the art. Although alkaline phosphatase may be used as commerically supplied, if the alkaline phosphatase contains significant amounts of ammonium ions, it is preferred that the alkaline phosphatase be purified prior to use in the preparation of the tracers of the present invention. One method for purifying the alkaline phosphatase involves passing the alkaline phosphatase over an ACA-44 (LKB) column (3 X 57 cm) using 0.2 M $Na_2CO_3$ (pH 9.4), 0.2 m$\underline{M}$, $MgCl_2$, and 0.1% deoxycholic acid, and assaying the fractions pooled and concentrated in an Amicon B15 minicon. Crude alkaline phosphatase-labeled steroid hormone glucuronides were purified in the following examples using minicolumns by loading 50 µℓ of the crude product on a Bio-Gel P2 gel (BIO-RAD, 100-200 mesh, 2 ml) packed in a polypropylene Econo-Column BIO-RAD, eluting the column with a buffer consisting of 0.1M sodium phosphate, 0.1mM $MgCl_2$ and 0.1% deoxycholic acid (pH 7.0) (600 µℓ), discarding the wash, and and eluting the purified alkaline phosphatase-labeled steroid hormone glucuronide with an additional 400 µℓ of the buffer. The progress of active ester formation was monitored by thin-layer chromatography employing silica gel plates (Merck 60 F-254) and $CH_2Cl_2$ to methanol ratios of either 9:1 or 8:2, the chromatograms sprayed with a mixture of cerric ammonium sulfate and sulfuric acid and then charred.

As previously mentioned, the above-noted tracers are useful as reagents in enzyme immunoassays and may be provided in dry powder form or in aqueous solution.

The following examples serve to illustrate the present invention and are not intended to be limitations on the disclosed invention.


EXAMPLE 1


Estradiol glucuronide tracers were prepared by forming active ester intermediates of estradiol glucuronide and reacting the esters *in situ* with alkaline phosphatase according to the following procedures:

A mixture comprising estradiol glucuronide sodium salt (20 mg, 42 µmol), N-hydroxysuccinimide (6 mg, 52 µmol), and 1-ethyl-3(3-dimethylaminopropyl)carbodiimide hydrochloride (8 mg, 42 µmol) was stirred in 1 ml of dry dimethylformamide for four hours to yield an estradiol glucuronide active ester. The estradiol glucuronide active ester was reacted *in situ* with 40 µℓ of purified alkaline phosphatase in molar ratios of 60:1, 120:1, 180:1, 240:1, 300:1, and 360:1 of active ester to alkaline phosphatase to yield a crude product. The crude product was purified over minicolumns to yield an alkaline phosphatase-labeled estradiol glucuronide tracer.

## EXAMPLE 2

A mixture comprising pregnanediol gluruconide (100 mg, 200 µmol) N-hydroxysuccinimide (30 mg, 260 µmol), 1-hydroxybenztriazole hydrate (0.1 mg, 0.75 µmol), and N,N'-dicyclohexylcarbodiimide (57 mg, 276 µmol) was stirred in 1 ml of dry dimethylformamide for one hour at 5° C, allowed to stand at room temperature for 18 hours, filtered and the filtrate evaporated under reduced pressure to yield a residue. The residue was dissolved in dry acetone, precipitated with ether, and the precipitate collected on a filter and dried to yield a pregnanediol glucuronide active ester as a white powder. 3.2 mg of the pregnanediol glucuronide active ester was dissolved in 200 µl of dry dimethyl formamide. Portions of the resulting solution in molar ratios of 120:1, 145:1, and 165:1 of active ester to alkaline phosphatase were mixed with 50 µl of purified alkaline phosphatase and shaken for 18 hours at 4° C to yield a crude product. The crude product was purified over minicolumns to yield an alkaline phosphatase-labeled pregnanediol glucuronide tracer.

## EXAMPLE 3

Pregnanediol glucuronide esters were formed and reacted *in situ* with alkaline phosphatase to form tracer preparations as follows:

Pregnanediol glucuronide (25.0 mg, 50 µmol), N-hydroxysuccinimide (8.5 mg, 74 µmol), and 1-ethyl-3(3-dimethylaminopropyl)carbodiimide hydrochloride (8.5 mg, 45 µmol) were stirred in 1 ml of dry dimethylformamide for 24 hours. A portion of the solution having a molar ratio of 490:1 of active ester to alkaline phosphatase was reacted with 400 µl of purified alkaline phosphatase to yield a crude product which was purified over 25 minicolumns and dialyzed against buffer consisting of 0.1M sodium phosphate, 0.1 mM $MgCl_2$ and 0.1% deoxycholic acid (pH 7.0) (2 x 500 ml) to yield an alkaline phosphatase-labeled pregnanediol glucuronide tracer.

As previously mentioned, the alkaline phosphatase labeled steroid hormone glucuronides of the present invention are useful as tracers in enzyme immunoassays.  The following examples serve to illustrate the use of tracers in enzyme immunoassays.

## EXAMPLE IV

### Estradiol Glucuronide Assay

Reagents: - estradiol glucuronide antibody solution comprising goat antiestradiol glucuronide antibody suspended in a carbonate buffer solution containing Triton X-301;
- alkaline phosphatase labeled-estradiol glucuronide tracer (prepared in Example II) suspended in a carbonate buffer containing protein, 1% lactose and 0.1% sodium azide;
- standard solutions of estradiol glucuronide, 0, 10, 20, 30 and 40 μM prepared by mixing appropriate amounts of estradiol glucuronide with charcoal stripped male urine containing 0.1% sodium azide;
- DAPS (double antibody precipitation suspension) comprising normal goat serum/porcine antigoat antiserum prepared according to Morgan and Lazarow, *Diabetes*, 12:115-126 (1963);
- Abbott A-Gent Alkaline Phosphatase Reagent
- quenching solution comprising 10 nM cystiene
- unassayed urine control

0114615

Procedure: Two hundred microliters (200 μl) of urine sample, standard solution or urine control are added to duplicate sets of culture tubes. A blank culture tube is also established by adding 25 μl of water to a separate culture tube. One hundred microliters (100 μl) of the buffered alkaline phosphatase-labeled estradiol glucuronide tracer solution is added to each culture tube except the blank tube, to which 100 μl of water is added. Known amounts of estradiol glucuronide antibody solution (100 μl) are added to all the culture tubes (including the blank), and each tube is vortexed to mix the samples thoroughly. After mixing, the tubes are covered and incubated at 37° C for ten minutes, whereupon 200 μl of DAPS is added to each culture tube. Each tube is again vortex-mixed thoroughly and incubated at 37° C for ten minutes. After incubation, all tubes are centrifuged at 1000 X g for ten minutes. One hundred microliter (100 μl) aliquots are removed from the supernate so as to not disturb the pellet in the bottom of each tube and then added to clean reaction tubes. To the supernate aliquot in the reaction tube is added 100 μl of Abbott A-Gent Alkaline Phosphatase Reagent, and the resulting mixture is mixed thoroughly and incubated at room temperature for 15 minutes. After the incubation, 1.5 ml of quenching solution is added to the mixture in each reaction tube and the absorbance of the resulting solution in each tube is read on a spectrophotometer at an appropriate wavelength for p-nitrophenol. The concentration of estradiol glucuronide in the sample is determined by comparing the absorbance measurement obtained for the sample with a standard curve prepared from the absorbance measurement obtained from the standards.

## EXAMPLE V

<u>Pregnanediol Glucuronide Assay</u>

Reagents: - pregnanediol glucuronide antibody solution comprising goat antipregnanediol glucuronide antibody suspended in a carbonate buffer solution containing Triton X-300;

- alkaline phosphatase labeled pregnanediol glucuronide tracer (prepared in Example III), suspended in a carbonate buffer containing protein, 1% lactose and 0.1% sodium azide;

- standard solutions of pregnanedriol glucuronide, 0, 10, 20, 30 and 40 µM prepared by mixing appropriate amounts of pregnanediol glucuronide with charcoal stripped male urine containing 0.1% sodium azide;

- DAPS (double antibody precipitation suspension) comprising normal goat serum/porcine antigoat antiserum prepared according to Morgan and Lazarow, <i>Diabetes</i>, 12:115-126, (1963);

- Abbott A-Gent Alkaline Phosphatase Reagent

- quenching solution comprising 10 nM cystine

- unassayed urine control.

Procedure: Twenty-five microliters (25 µl) of urine sample, standard solution or urine control are added to duplicate sets of culture tubes. A blank culture tube is also established by adding 25 µl of water to a separate culture tube. One hundred microliters (100 µl) of the buffered alkaline phosphatase-labeled pregnanediol glucuronide solution is added to each culture tube except the blank tube, to which 100 µl of water is added. Known amounts of pregnanediol glucuronide antibody solution (100 µl) are added to all the culture tubes (including the blank), and each tube is vortexed to mix the samples thoroughly. After mixing, the tubes are covered and incubated at 37° C for ten minutes, whereupon 200 µℓ of DAPS is added to each culture tube. The tubes are again vortex-mixed thoroughly and incubated at 37° C for ten minutes. After incubation, all tubes are centrifuged at 1000 X g for ten minutes. One hundred microliter (100 µℓ) aliquots are removed from the supernate in each tube and carefully pipetted into labeled reaction tubes so as not to disturb the pellet in the bottom of each tube and then added to a clean reaction tube. To the supernate aliquot in each reaction tube is added 100 µℓ of Abbott A-Gent Alkaline Phosphatase Reagent, and the resulting mixture is mixed thoroughly and incubated at room temperature for 15 minutes. After the incubation, 1.5 ml of quenching solution is added to the mixture in each reaction tube and the absorbance of the solution in each tube is read on a spectrophotometer at an appropriate wavelength for p-nitrophenol. The concentration of pregnanediol glucuronide in the samples is determined by comparing the absorbance measurement obtained for the sample with a standard curve prepared from the absorbance measurements obtained from the standards.

Although this invention has been described with respect to specific modifications, the details thereof are not to be construed as limitations, for it will be apparent that various equivalents, changes and modifications may be resorted to without departing from the spirit and scope thereof and it is understood that such equivalent embodiments are intended to be included herein.

CLAIMS:

1. A compound useful as a tracer in enzyme immunoassays comprising a steroid hormone glucuronide covalently bound to alkaline phosphatase through an amide bond formed between a carboxyl group of the steroid hormone glucuronide and an amine group of alkaline phosphatase.

2. A compound according to Claim 1 wherein the steroid hormone glucuronide is pregnanediol glucuronide.

3. A compound according to Claim 1 wherein the steroid hormone glucuronide is estradiol glucuronide.

4. A compound according to Claim 1 wherein the steroid hormone glucuronide is estriol glucuronide.

5. A compound according to Claim 1 wherein the steroid hormone glucuronide is estrone glucuronide.